# FASCICULE DE BREVET EUROPEEN

(11) **EP 1 556 699 B1**
(45) Date de publication et mention de la délivrance du brevet: **25.03.2009**
(21) Numéro de dépôt: 03778473.3
(22) Date de dépôt: 28.10.2003
(51) Int. Cl.: G01N 33/574

(54) **PROCEDE DE DOSAGE DU NGF POUR LE DIAGNOSTIC IN VITRO DU CANCER DU SEIN ET UTILISATION EN THERAPIE**
VERFAHREN ZUR DOSIERUNG VON NGF ZUR IN VITRO DIAGNOSE VON BRUSTKREBS UND THERAPEUTISCHE VERWENDUNG
METHOD FOR NGF ASSAY FOR IN VITRO DIAGNOSIS OF BREAST CANCER AND THERAPEUTIC USE

(30) Priorité: 28.10.2002 FR 0213428
(43) Date de publication de la demande: 27.07.2005
(73) Titulaire: BIOMERIEUX, 69280 Marcy-L'Etoile (FR); UNIVERSITE DES SCIENCES ET TECHNOLOGIES DE LILLE, 59655 Villeneuve-d'Ascq Cedex (FR)
(72) Inventeur: ADRIAENSSENS, Eric, F-59227 Saulzoir (FR); CHOQUET-KASTYLEVSKY, Geneviève, F-69500 Bron (FR); DOLLE, Laurent, F-59650 Villeneuve D'Ascq (FR); EL-YAZIDI, Belkoura, Ikram, F-59650 Villeneuve D'Ascq (FR); HONDERMARCK, Hubert, F-59650 Villeneuve D'Ascq (FR)
(86) Numéro de dépôt international: PCT/FR2003/003193
(87) Numéro de publication internationale: WO 2004/040312

(56) Documents cités:
- WO-A-01/64247
- WO-A-94/06935
- US-A- 6 008 003
- DESCAMPS S. ET AL.: "Nerve growth factor is mitogenic for cancerous but not normal human breast epithelial cells." J. BIOL. CHEM., vol. 273, no. 27, 3 juillet 1998 (1998-07-03), pages 16659-16662, XP002277430
- TAGLIABUE E. ET AL.: "Nerve growth factor cooperates with p185HER2 in activating growth of human breast carcinoma cells." J. BIOL. CHEM., vol. 275, no. 8, 25 février 2000 (2000-02-25), pages 5388-5394, XP002238474
- DESCAMPS S. ET AL.: "Nerve growth factor stimulates proliferation and survival of human breast cancer cells through two distinct signaling pathways." J. BIOL. CHEM., vol. 276, no. 21, 25 mai 2001 (2001-05-25), pages 17864-17870, XP002238475 cité dans la demande
- DESCAMPS S. ET AL.: "Expression of nerve growth factor receptors and their prognostic value in human breast cancer." CANCER RES., vol. 61, 1 juin 2001 (2001-06-01), pages 4337-4340, XP002238476
- DOLLE L. ET AL.: "Nerve growth factor overexpression and autocrine loop in breast cancer cells." ONCOGENE, vol. 22, 28 août 2003 (2003-08-28), pages 5592-5601, XP002277431

## Description

La présente invention concerne le domaine de la cancérologie. Plus particulièrement, la présente invention a pour objet un procédé de diagnostic du cancer du sein chez un patient humain par détermination de la présence du facteur de croissance nerveux (NGF) dans un échantillon biologique issu de ce patient, ledit procédé pouvant être utilisé tant dans le diagnostic précoce, le dépistage, le suivi thérapeutique, le pronostic, que dans le diagnostic des rechutes dans le cadre du cancer du sein. De plus, du fait de la capacité des cellules du cancer du sein à produire du NGF, la présente invention concerne également la thérapie.

Chez la femme, le cancer du sein est la première cause de mortalité par cancer dans les pays industrialisés. Il est estimé que la taille minimale d'une tumeur repérable par mammographie est de 1 cm. Les cancers du sein se développent lentement. Néanmoins, cette tumeur de petite taille présente un passé évolutif de 8 ans en moyenne lors du diagnostic. L'étiologie du cancer du sein n'est pas bien définie. Des prédispositions familiales ont été mises en évidence. L'âge est le facteur de risque le plus important. Ainsi, le risque augmente de 0,5% par année d'âge dans les pays occidentaux. D'autres facteurs de risques sont connus tels que le nombre des grossesses et l'âge de la première grossesse, l'allaitement, l'âge de la puberté et de la ménopause, les traitements oestrogéniques après la survenue de la ménopause, le stress et la nutrition.

Le test disponible et utilisé en dépistage de masse pour le cancer du sein est une technique d'imagerie : la mammographie. Grâce à cette technique, la mortalité due aux cancers du sein a fortement diminué (30% de réduction de mortalité), ce qui souligne l'importance du dépistage des tumeurs en terme de santé publique. Néanmoins, les techniques de dépistage souffrent d'un certain nombre de handicaps. La mammographie nécessite un matériel performant et du personnel qualifié ce qui est coûteux dans le cadre d'un dépistage de masse.

En pratique clinique, la caractérisation d'une tumeur en terme de malignité est réalisée après sa découverte par des méthodes histologiques dans des laboratoires spécialisés. Un ensemble de paramètres tels que la taille de la tumeur, son grade histopathologique, l'inflammation associée et l'invasion ganglionnaire sont utilisés pour décider de la thérapeutique et pour estimer le pronostic de la maladie.

Des marqueurs qui permettent de distinguer les cellules tumorales des cellules saines sont recherchés et étudiés depuis des années pour le cancer du sein. Ils permettraient de diagnostiquer précocement la maladie, d'en établir le pronostic et la sensibilité au traitement, et d'en surveiller l'évolution. Jusqu'à présent les marqueurs candidats qui ont été identifiés et étudiés ont été des oncogènes, des marqueurs tissulaires et des marqueurs associés à l'angiogénèse ou aux capacités métastatiques de la tumeur. Actuellement les marqueurs de cancer du sein identifiés servent principalement pour le suivi thérapeutique. Il n'existe pas de test biologique validé pour le diagnostic précoce, ni pour le dépistage du cancer du sein. Seule la détection des récepteurs aux oestrogènes sur le tissu tumoral permet de déterminer si les tumeurs seront ou non hormono-sensibles.

Un nombre limité de marqueurs antigéniques, en particulier le CA 15-3 (Basuyau, J. P., M. P. Blanc-Vincent, J. M. Bidart, A. Daver, L. Deneux, N. Eche, G. Gory-Delabaere, M. F. Pichon, and J. M. Riedinger. 2000. [Standards, Options and Recommendations (SOR) for tumor markers in breast cancer. SOR Working Group]. Bull Cancer. 87:723-37.) a été identifié dans le cas des cellules mammaires cancéreuses. Ce marqueur est utilisé en pratique courante pour le suivi des patientes, en particulier pour la détection de récidive, mais, en raison de sa faible sensibilité, il n'est pas proposé en test de dépistage ni de diagnostic.

Depuis plusieurs années des travaux portant sur les antigènes associés au cancer du sein ont été développés non pas pour rechercher des marqueurs, mais pour rechercher des cibles pour une immunothérapie. Ceux-ci vont de la mise en évidence d'une immunité humorale contre les antigènes T/Tn (Springer, G. F. 1997. Immunoreactive T and Tn epitopes in cancer diagnosis, prognosis, and immunotherapy. J Mol Med. 75:594-602.), jusqu'à la découverte plus récente d'anticorps et de réponses des cellules T dirigés contre p53 (Gnjatic, S., Z. Cai, M. Viguier, S. Chouaib, J. G. Guillet, and J. Choppin. 1998. Accumulation of the p53 protein allows recognition by human CTL of a wild-type p53 epitope presented by breast carcinomas and melanomas. J Immunol. 160:328-33) et HER-2/neu (Disis, M. L., and M. A. Cheever. 1997. HER-2/neu protein: a target for antigen-specific immunotherapy of human cancer. Adv Cancer Res. 71:343-71).

Plus récemment, une série de nouveaux antigènes potentiels a été mise en évidence par l'approche SEREX (serological expression cloning), reposant sur la construction de librairies d'ADNc de cellules tumorales et un screening par le sérum autologue. Un screening sérologique de librairie de cancer du sein a ainsi permis la mise en évidence de l'antigène *ING1* (Jager, D., E. Stockert, M. J. Scanlan, A. O. Gure, E. Jager, A. Knuth, L. J. Old, and Y. T. Chen. 1999. Cancer-testis antigens and ING1 tumor suppressor gene product are breast cancer antigens: characterization of tissue-specific ING1 transcripts and a homologue gene. Cancer Res. 59:6197-204.), puis d'un nouvel antigène de différenciation *NY-BR-1*, exprimé selon les auteurs dans 80% des cancers du sein et induisant la production d'anticorps IgG chez les patientes (Jager, D., E. Stockert, A. O. Gure, M. J. Scanlan, J. Karbach, E. Jager, A. Knuth, L. J. Old, and Y. T. Chen. 2001. Identification of a tissue-specific putative transcription factor in breast tissue by serological screening of a breast cancer library. Cancer Res. 61:2055-61). Ce type d'approche, qui a principalement servi à la recherche de cibles potentiellement utilisables pour le développement de vaccins, n'exclut pas *a priori* les antigènes présents dans le tissu normal (c'est le cas de *NY-BR-1),* ni ceux reconnus par un nombre limité de sérums de patientes (2/14 pour *ING1),* ils ne sont donc pas exploitables pour une stratégie de dépistage ou de diagnostic précoce. Par la même approche, d'autres antigènes induisant une réponse immunitaire humorale chez des patientes ont été signalés comme NY-BR-62, NY-BR-85 et la protéine D52. Ces antigènes seraient surexprimés respectivement dans 60%, 90% et 60% des cancers du sein (Scanlan, M. J., and D. Jager. 2001. Challenges to the development of antigen-specific breast cancer vaccines. Breast Cancer Res. 3:95-8.).

Les phénomènes moléculaires conduisant au développement d'un cancer du sein impliquent des modifications de la structure et de l'expression d'oncogènes (tel que ras) et de gènes suppresseurs de tumeurs comme p53. La croissance des cellules tumorales dans la plupart des cancers du sein est dépendante des hormones oestrogéniques (oestradiol et progestérone) et de facteurs de croissance qui contrôlent la prolifération, la migration et l'apoptose. Ces facteurs de croissance soit stimulent, soit inhibent la prolifération, migration et différentiation des cellules tumorales de manière à agir de concert pour favoriser la croissance du cancer et les métastases. Par exemple, les facteurs de croissance de type insuline, le facteur de croissance de transformation α (TGF-α) et les facteurs de croissance du fibroblaste (FGF) peuvent tous stimuler la prolifération des cellules du cancer du sein, tandis que l'inhibiteur du facteur de croissance dérivé du sein (MDGI) et le facteur de croissance de transformation β (TGF-β) inhibent leur croissance.

Descamps, S. et al. ont démontré que le facteur de croissance NGF, le premier facteur neurotrophique découvert, ajouté *in vitro* à des lignées cellulaires en culture (NGF exogène), est potentiellement capable d'activer à la fois la survie et la prolifération des cellules du cancer du sein (1998, J. Biol. Chem., 273(27), 16659-16662 et 2001, J. Biol. Chem., 276(21), 17864-17870).

Le NGF a été isolé à l'origine pour sa capacité à stimuler tant la survie que la différentiation des neurones périphériques, devenant plus tard l'élément archétype de la famille neurotrophique des polypeptides (Levi-Montalcini, R., 1987, Science, 4237, 1154-1162). Une fonction biologique majeure du NGF est le maintien et la survie des neurones post-mitotiques, ce qui fait de lui un candidat important pour le traitement des maladies neurodégénératives. Outre son rôle dans le développement et le maintien des cellules neuronales, le NGF a également des effets significatifs sur les cellules non neuronales. Ainsi, par exemple, il est un facteur de survie autocrine des lymphocytes B (Torcia, M., et al., 1996, Cell, 85, 345-356).

Le NGF génère des signaux intracellulaires par interaction avec deux classes de récepteur de membrane : le produit proto-oncogénique de TrkA p140^{trkA}, qui possède une activité tyrosine kinase intrinsèque, et un deuxième récepteur, p75^{NTR}, qui appartient à la famille des récepteurs du facteur de nécrose tumorale (TNF). Dans les cellules du cancer du sein, il existe deux voies de signalisation distinctes activées par le NGF. L'effet mitogénique nécessite le récepteur p140^{trkA} et la cascade MAP kinase, tandis que l'effet anti-apoptose dépend du récepteur p75^{NTR} et de l'activation en aval du facteur NF-κB (Descamps, S. et al., 2001, *supra*). Ainsi, pour le diagnostic du cancer du sein, certains auteurs se sont tournés vers la détection des récepteurs au NGF, comme par exemple dans la demande de brevet WO94/06935 qui décrit un procédé de diagnostic d'un état précancéreux ou cancéreux chez un patient par dosage de la quantité du récepteur p75.

L'effet biologique puissant du NGF sur les cellules du cancer du sein soulève également la question de sa distribution dans la glande mammaire et de son origine. On pense généralement que le NGF est produit par des cibles de l'innervation sympathique et sensorielle, bien qu'il n'ait pas été trouvé dans la circulation sanguine (Bonini, S. et al ; 1996, Proc., Natl. Acad. Sci. USA, 93,10955-10960).

La demanderesse a maintenant mis en évidence de façon surprenante que les cellules du cancer du sein produisent elles-mêmes du NGF, tandis que des cellules épithéliales mammaires normales correspondantes n'en produisent pas, de sorte que le NGF peut être utilisé à titre de marqueur tumoral ou bien à titre de cible thérapeutique.

Ainsi, la présence invention a pour premier objet un procédé de diagnostic du cancer du sein par détermination de la présence de NGF dans des échantillons biologiques issus de patients suspectés d'être atteints du cancer du sein.

Le procédé de l'invention permet donc de diagnostiquer le cancer du sein par un test simple consistant à rechercher la présence de NGF dans un échantillon biologique prélevé d'un patient. La demanderesse a montré de façon inattendue que des cellules cancéreuses produisaient du NGF, tandis que les cellules correspondantes non cancéreuses en étaient incapables, comme cela sera mis en évidence de façon plus détaillée ci-après. Ainsi, la détermination de la présence de NGF dans l'échantillon permet de conclure à la pathologie recherchée, l'absence de NGF permettant de conclure à l'absence de pathologie.

On entend par détermination de la présence de NGF soit la détection directe du NGF, soit la culture de cellules sensibles au NGF, soit la détection de l'ARNm du NGF dans l'échantillon biologique, ou tout autre procédé de détermination de la présence d'une protéine dans un échantillon, connu de l'homme du métier.

La détermination de la présence de NGF par détection directe du NGF constitue un mode de réalisation particulier de l'invention.

Par détection directe du NGF, on entend la mise en évidence du NGF lui-même dans l'échantillon biologique.

La détection directe du NGF dans l'échantillon biologique peut être mise en oeuvre par tout moyen connu de l'homme du métier, comme par exemple par test immunologique ou par spectrométrie de masse, ce qui constitue un mode de réalisation particulier de l'invention.

Le test immunologique peut être tout test largement connu de l'homme du métier impliquant des réactions immunologiques, à savoir des réactions entre le NGF et un partenaire de liaison spécifique du NGF.

Les partenaires de liaison spécifiques du NGF sont tout partenaire susceptible de se lier au NGF. A titre d'exemple, on peut citer les anticorps, les fractions d'anticorps, les récepteurs et toute autre protéine capable de se lier au NGF.

Les anticorps partenaires de liaison sont soit des anticorps polyclonaux, soit des anticorps monoclonaux.

Les anticorps polyclonaux peuvent être obtenus par immunisation d'un animal avec du NGF, suivie de la récupération des anticorps recherchés sous forme purifiée, par prélèvement du sérum dudit animal, et séparation desdits anticorps des autres constituants du sérum, notamment par chromatographie d'affinité sur une colonne sur laquelle est fixée un antigène spécifiquement reconnu par les anticorps, notamment le NGF.

Les anticorps monoclonaux peuvent être obtenus par la technique des hybridomes dont le principe général est rappelé ci-après.

Dans un premier temps, on immunise un animal, généralement une souris, (ou des cellules en culture dans le cadre d'immunisations *in vitro)* avec du NGF, dont les lymphocytes B sont alors capables de produire des anticorps contre ledit antigène. Ces lymphocytes producteurs d'anticorps sont ensuite fusionnés avec des cellules myélomateuses "immortelles" (murines dans l'exemple) pour donner lieu à des hybridomes. A partir du mélange hétérogène des cellules ainsi obtenu, on effectue alors une sélection des cellules capables de produire un anticorps particulier et de se multiplier indéfiniment. Chaque hybridome est multiplié sous la forme de clone, chacun conduisant à la production d'un anticorps monoclonal dont les propriétés de reconnaissance vis-à-vis de l'antigène tumoral d'intérêt pourront être testées par exemple en ELISA, par immunotransfert en une ou deux dimensions, en immunofluorescence, ou à l'aide d'un biocapteur. Les anticorps monoclonaux ainsi sélectionnés, sont par la suite purifiés notamment selon la technique de chromatographie d'affinité décrite ci-dessus.

Des exemples d'anticorps anti-NGF sont connus et sont disponibles notamment dans le catalogue de R&D Systems ou de Santa Cruz.

Les partenaires de liaison spécifiques du NGF pourront être marqués pour la révélation de la liaison NGF/partenaire de liaison lorsque le partenaire de liaison est utilisé comme réactif de détection, et donc pour la détection directe du NGF dans l'échantillon biologique.

Par marquage des partenaires de liaison, on entend la fixation d'un marqueur capable de générer directement ou indirectement un signal détectable. Une liste non limitative de ces marqueurs consiste en :
- les enzymes qui produisent un signal détectable par exemple par colorimétrie, fluorescence, luminescence, comme la péroxydase de raifort, la phosphatase alcaline, la α-galactosidase, la glucose-6-phosphate déshydrogénase,
- les chromophores comme les composés fluorescents, luminescents, colorants,
- les molécules radioactives comme le ³²P, le ³⁵S ou le ¹²⁵I , et
- les molécules fluorescentes telles que l'alexa ou les phycocyanines.

Des systèmes indirects peuvent être aussi utilisés, comme par exemple des ligands capables de réagir avec un anti-ligand. Les couples ligand/anti-ligand sont bien connus de l'homme du métier, ce qui est le cas par exemple des couples suivants : biotine/streptavidine, haptène/anticorps, antigène/anticorps, peptide/anticorps, sucre/lectine, polynucléotide/complémentaire du polynucléotide. Dans ce cas, c'est le ligand qui porte l'agent de liaison. L'anti-ligand peut être détectable directement par les marqueurs décrits au paragraphe précédent ou être lui-même détectable par un ligand/anti-ligand.

Ces systèmes indirects de détection peuvent conduire, dans certaines conditions, à une amplification du signal. Cette technique d'amplification du signal est bien connue de l'homme du métier, et l'on pourra se reporter aux demandes de brevet antérieures FR98/10084 ou WO-A-95/08000 de la demanderesse ou à l'article J. Histochem. Cytochem. 45 : 481-491, 1997.

Selon le type de marquage du conjugué utilisé, l'homme du métier ajoutera des réactifs permettant la visualisation du marquage.

A tire d'exemple de tests immunologiques tels que définis ci-dessus, on peut citer les méthodes « sandwich » telles qu'ELISA, IRMA et RIA, les méthodes dites de compétition et les méthodes d'immunodétection directe comme l'immunohistochimie, l'immunocytochimie, le Western-blot et le Dot-blot.

La spectrométrie de masse peut également être utilisée pour la détection directe du NGF dans l'échantillon biologique. Le principe de la spectrométrie est largement connu de l'homme du métier et est décrit par exemple dans Patterson, S., 2000, Mass spectrometry and proteomics. Physiological Genomics 2, 59-65.

Pour ce faire, l'échantillon biologique préalablement traité ou non est passé dans un spectromètre de masse et on compare le spectre obtenu avec celui du NGF. Un exemple de traitement préalable de l'échantillon consiste à le faire passer sur un support d'immunocapture, comportant un des partenaires de liaison du NGF, par exemple un anticorps dirigé contre le NGF.

L'échantillon biologique utilisé pour la détection directe du NGF, susceptible de contenir du NGF en tant que tel, peut être constitué par du fluide biologique ou un tissu provenant de la biopsie de la tumeur ou des métastases du patient considéré.

A titre de fluide biologique, on peut citer le sang, la moelle osseuse, le lait, le liquide céphalo-rachidien, les urines et les épanchements.

Pour la détection du NGF, le fluide biologique, qui constitue un mode de réalisation particulier de l'invention, peut nécessiter un traitement particulier. En effet, le fluide biologique peut contenir le NGF en tant que tel, ou bien il peut contenir des cellules tumorales circulantes qui contiennent du NGF, et éventuellement des cellules tumorales circulantes qui sont capables de sécréter le NGF.

Ainsi, selon un mode de réalisation de l'invention, le fluide biologique est préalablement traité pour isoler les cellules tumorales circulantes contenues dans le dit fluide.

Par isoler les cellules tumorales circulantes, on entend obtenir une fraction cellulaire enrichie en cellules tumorales circulantes.

Le traitement du fluide pour isoler les cellules tumorales circulantes peut être effectué par tri cellulaire dans un cytomètre de flux, par enrichissement sur Ficoll, par enrichissement par billes magnétiques recouvertes d'anticorps spécifiques, ou par toute autre méthode d'enrichissement spécifique connue de l'homme du métier.

Dans le cas du sang ou de la moelle à titre de fluide biologique, les cellules tumorales circulantes peuvent être isolées grâce à une technique de séparation cellulaire sur Ficoll associée à une déplétion des cellules sanguines utilisant des anticorps anti-CD45 couplés à des billes magnétiques (Dynal Biotech ASA, Norvège).

La détection directe du NGF peut alors être effectuée directement à partir de cellules tumorales circulantes isolées du fluide biologique, par exemple par marquage immunocytochimique de ces cellules avec un anticorps anti-NGF, après avoir déposé les cellules tumorales circulantes sur une lame par cytospin. La détection directe du NGF peut également être effectuée directement dans les cellules tumorales circulantes en utilisant la méthode de cytométrie de flux telle que décrite dans Métézeau P, Ronot X, Le Noan-Merdrignac G, Ratinaud MH, La cytométrie en flux pour l'étude de la cellule normale ou pathologique (Tome I), Eds Medsi-MacGrawhill.

Dans ces conditions, lesdites cellules circulantes peuvent être traitées dans des conditions permettant le blocage du NGF à l'intérieur desdites cellules. Un tel traitement est décrit par exemple dans Intracellular Flow Cytometry, Applied reagents and Techniques, pp 1-21, BD Pharmingen.

La détection du NGF se fait alors après avoir rendu perméable la membrane des cellules pour faire rentrer les partenaires de liaison spécifique du NGF.

La détection directe du NGF à partir des cellules circulantes peut également être effectuée à l'aide du procédé décrit dans la demande de brevet WO03/076942 déposée par l'une des Demanderesses.

La détection directe du NGF dans les cellules tumorales peut encore être effectuée dans le milieu de culture desdites cellules après les avoir cultivées dans des conditions telles qu'elles sécrètent du NGF.

Ces conditions de culture sont des conditions classiques telles que 37°C sous atmosphère humide et à 5% de CO₂.

Lorsque l'échantillon biologique à tester est du tissu provenant de la biopsie de la tumeur ou des métastases du patient, ce qui constitue un mode de réalisation particulier de l'invention, la détection directe du NGF est effectuée directement sur les coupes obtenues, sans traitement préalable dudit tissu.

Un autre mode de détection de la présence du NGF consiste en la culture, en présence de l'échantillon biologique, de cellules sensibles au NGF, ce qui constitue un mode de réalisation particulier de l'invention.

Dans ce cas, la détection de la présence du NGF dans un échantillon biologique est mise en évidence par la réaction des cellules sensibles au NGF.

Par cellules sensibles au NGF, on entend toute cellule stimulée en présence de NGF (croissance, apotose, etc.).

A titre de cellules sensibles au NGF, on peut citer les cellules phéochromocytomes PC12 (Hondermarck H. et al., 1994, Proc. Natl. Acad. Sci., 91:9377-81) et les cellules embryonnaires d'origine neuronale (Hattori A., et al., 1993, J. Biol. Chem., 268 :2577-2582).

L'échantillon biologique que l'on peut utiliser pour la détection de la présence du NGF par culture de cellules sensibles au NGF peut être tout échantillon tel que décrit précédemment.

Ainsi, l'échantillon biologique peut être constitué de fluide biologique, le cas échéant préalablement traité pour isoler les cellules tumorales circulantes, elles-mêmes pouvant ensuite être cultivées en conditions telles qu'elles sécrètent du NGF, comme décrit précédemment.

Un autre mode de détection de la présence de NGF dans les échantillons biologiques consiste en la détection de l'ARNm du NGF dans ledit échantillon, ce qui constitue un autre mode de réalisation de l'invention.

La détection d'ARNm dans un échantillon liquide est largement connue de l'homme du métier.

Elle peut par exemple être mise en oeuvre par des réactions d'hybridation entre l'ARNm cible et un acide nucléique capable de liaison avec l'ARNm cible.

On entend par acide nucléique les oligonucléotides, les acides désoxyribonucléiques et les acides ribonucléiques, ainsi que leurs dérivés.

Le terme « oligonucléotide » désigne un enchaînement d'au moins 2 nucléotides (désoxyribonucléotides ou ribonucléotides, ou les deux), naturels ou modifiés, susceptibles de s'hybrider, dans des conditions appropriées d'hybridation, avec un oligonucléotide au moins partiellement complémentaire. Par nucléotide modifié, on entend par exemple un nucléotide comportant une base modifiée et/ou comportant une modification au niveau de la liaison internucléotidique et/ou au niveau du squelette. A titre d'exemple de base modifiée, on peut citer l'inosine, la méthyl-5-désoxycytidine, la diméthylamino-5-désoxyuridine, la diamino-2,6-purine et la bromo-5-désoxyuridine. Pour illustrer une liaison internucléotidique modifiée, on peut mentionner les liaisons phosphorothioate, N-alkylphosphoramidate, alkylphosphonate et alkylphosphodiester. Les alpha-oligonucléotides tels que ceux décrits dans FR-A-2 607 507, les LNA tels que phosphorothioate-LNA and 2'-thio-LNA décrits dans Bioorganic & Medicinal Chemistry Letters, Volume 8, Issue 16, 18 August 1998 ,pages 2219-2222, et les PNA qui font l'objet de l'article de M. Egholm et al., J. Am. Chem. Soc. (1992), 114, 1895-1897, sont des exemples d'oligonucléotides constitués de nucléotides dont le squelette est modifié.

La révélation des réactions d'hybridation peut être effectuée par marquage des acides nucléiques de liaison, comme illustré précédemment.

Avant hybridation avec l'acide nucléique de liaison, l'ARNm cible peut être extrait par des méthodes connues de l'homme du métier, puis éventuellement amplifié, comme par exemple par RT-PCR ou par NASBA (Maleck, L., et al., 1994, Methods in Molecular Biology, 28, ch 36, Ed P.G. Isaac, Humana Press, Inc., Totowa, NJ).

L'échantillon biologique que l'on peut utiliser pour la détection de la présence du NGF par détection d'ARNm du NGF peut être tout échantillon tel que décrit précédemment.

Ainsi, l'échantillon biologique peut être constitué de tissu issu de biopsie de tumeur ou de métastase, ou bien de fluide biologique, le cas échéant préalablement traité pour isoler les cellules tumorales circulantes, comme décrit précédemment.

Le procédé de l'invention peut être utilisé tant pour le diagnostic précoce, que pour le dépistage, le suivi thérapeutique, le pronostic et le diagnostic des rechutes dans le cadre du cancer du sein puisque seules les cellules cancéreuses produisent du NGF et que cette production est fonction du grade du cancer.

Ainsi, un autre objet de l'invention consiste en l'utilisation du procédé de l'invention dans le diagnostic précoce, le dépistage, le suivi thérapeutique, le pronostic et le diagnostic des rechutes dans le cadre du cancer du sein.

Outre un rôle de marqueur tumoral, le NGF peut également avoir un rôle de cible thérapeutique. En effet, du fait de la capacité des cellules du cancer du sein à produire du NGF, tandis que les cellules normales n'en produisent pas, la prolifération tumorale et la dissémination à distance des cellules du cancer du sein peuvent être bloquées par un partenaire de liaison du NGF susceptible de bloquer la prolifération cellulaire ou par un inhibiteur du NGF.

Les partenaires de liaison du NGF et les inhibiteurs du NGF peuvent donc être utilisés comme médicaments.

Ainsi, la présente invention a également pour objet l'utilisation d'un partenaire de liaison du NGF ou d'un inhibiteur du NGF pour la préparation d'un médicament, étant entendu que, dans le cadre du cancer, les partenaires de liaison du NGF ne sont pas des récepteurs pour le NGF ou des fragments actifs de ces récepteurs.

Selon un mode de réalisation particulier de l'invention, ledit médicament est utile pour bloquer la prolifération tumorale et la dissémination à distance chez les patients atteints du cancer du sein.

Les compositions pharmaceutiques comprenant à titre de principe actif au moins un partenaire de liaison du NGF ou un inhibiteur du NGF, éventuellement en association avec un excipient pharmaceutiquement acceptable, sont également incluses dans l'invention, étant entendu que, dans le cadre du cancer, les partenaires de liaison du NGF ne sont pas des récepteurs pour le NGF ou des fragments actifs de ces récepteurs.

Les compositions pharmaceutiques utiles contre le cancer du sein comprennent, à titre de principe actif, au moins un partenaire de liaison du NGF susceptible de bloquer la prolifération tumorale cellulaire ou un inhibiteur du NGF.

Les partenaires de liaison du NGF spécifiques appropriés à titre de principe actif sont notamment tels que définis précédemment dans les tests immunologiques et peuvent être tout autre partenaire connu de l'homme du métier capable de bloquer la prolifération tumorale. Selon un mode de réalisation particulier, le partenaire spécifique susceptible de bloquer la prolifération de cellules de cancer du sein est un anticorps anti-NGF.

Par inhibiteur du NGF, on entend les inhibiteurs directs du NGF, c'est-à-dire les inhibiteurs bloquant l'activité biologique de la protéine, les inhibiteurs des voies d'exocytose du NGF, les inhibiteurs de l'ARNm du NGF et les inhibiteurs du gène codant pour le NGF.

A titre d'inhibiteur direct du NGF, on peut citer l'inhibiteur K-252a (Tapley, P., et al., 1992, Oncogene 7, 371-381).

Les inhibiteurs des voies d'exocytose des protéines et en particulier du NGF sont largement connus de l'homme du métier.

A titre d'inhibiteur de l'ARNm du NGF, on peut utiliser un fragment synthétique de cet ARNm. En effet, la technologie du ARN interférence est basée sur l'utilisation d'un oligonucléotide ARN double brin correspondant à une courte séquence de l'ARNm cellulaire à inhiber. Cet oligonucléotide en duplex (introduit ou non dans un plasmide), après entrée dans la cellule, est pris en charge par le système enzymatique Dicer/Risc, ce qui va entraîner la dégradation de l'ARNm cellulaire correspondant (Dykxhoorn D et al., 2003, Nature Reviews, vol. 4, p457-467).

A titre d'inhibiteur du gène codant pour le NGF, on peut citer un oligonucléotide anti-sens du NGF. Cet oligonucléotide peut être facilement préparé par l'homme du métier.

Pour cibler l'action thérapeutique des partenaires de liaison du NGF ou des différents inhibiteurs, ces derniers peuvent être mis en conditions telles qu'ils pénètrent spécifiquement dans les cellules d'intérêt, telles que les cellules tumorales, ce qui constitue un autre mode de réalisation de l'invention.

A cet effet, ils peuvent par exemple être fixés à un partenaire qui permet une telle pénétration, comme par exemple une molécule porteuse, un polymère tel que les polymères utilisés en thérapie génique ou bien un vecteur viral tel que les adénovirus et les poxvirus, également utilisés en thérapie génique.

Par exemple, dans le cadre du cancer du sein, la molécule porteuse peut être un anticorps anti-MUCI ou un anticorps anti-cellule épithéliale, ou bien encore un anticorps anti-HER-2/neu.

De préférence, lorsque la composition pharmaceutique comprend à titre de principe actif un partenaire de liaison du NGF ou un inhibiteur du NGF tel qu'un ihnibiteur direct ou un inhibiteur des voies d'exocytose du NGF, ces derniers sont mis en conditions telles qu'ils pénètrent spécifiquement dans les cellules tumorales d'intérêt, les inhibiteurs de l'ARNm du NGF et les inhibiteurs du gène codant pour le NGF ayant la capacité de pénétrer naturellement dans lesdites cellules.

La quantité et la nature de l'excipient peuvent être facilement déterminées par l'homme du métier. Elles sont choisies selon la forme pharmaceutique et le mode d'administration souhaités.

Dans les compositions pharmaceutiques de l'invention pour l'administration orale, sublinguale, sous-cutanée, intramusculaire, intratumorale, intraveineuse, topique, locale, intratrachéale, intranasale, transdermique, rectale, intraoculaire, intra-auriculaire, ledit principe actif pouvant être administré sous forme unitaire d'administration.

Les formes unitaires d'administration peuvent être par exemple des comprimés, des gélules, des granules, des poudres, des solutions ou suspensions orales injectables, des timbres transdermiques (« patch »), des formes d'administration sublinguale, buccale, intratrachéale, intraoculaire, intranasale, intra-auriculaire, par inhalation, des formes d'administration topique, transdermique, sous-cutanée, intramusculaire, intratumorale ou intraveineuse, des formes d'administration rectale ou des implants. Pour l'administration topique, on peut envisager des crèmes, gels, pommades, lotions ou collyres.

Ces formes galéniques sont préparées selon les méthodes usuelles des domaines considérés.

Lesdites formes unitaires sont dosées pour permettre une administration journalière de 0,001 à 10 mg de principe actif par kg de poids corporel, selon la forme galénique.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse du patient.

Selon un autre mode de réalisation, la présente invention concerne également une méthode de traitement du cancer du sein qui comprend l'administration, à un patient, d'une dose efficace d'un partenaire de liaison du NGF ou d'un inhibiteur du NGF.

L'invention sera mieux comprise à l'aide des exemples suivants donnés à titre illustratif et non limitatif, ainsi qu'à l'aide des figures 1 à 5 annexées, sur lesquelles :
- la figure 1 concerne des représentations graphiques relatives à la mise en évidence de l'expression du NGF dans des cellules cancéreuses, dans laquelle :
   - la figure 1A représente un gel d'électrophorèse mettant en évidence, après RT-PCR, la sécrétion du NGF par les cellules cancéreuses MCF-7, T47D, MDA-MB-231 et BT20 et par les cellules de la glande submaxillaire à titre de témoin, ainsi que l'absence d'une telle sécrétion par les cellules normales NBEC (Normal Breast Epithelial Cells),
   - la figure 1B représente un gel d'électrophorèse mettant en évidence, après immunoblotting, la sécrétion du NGF par les cellules cancéreuses MCF-7, T47D, MDA-MB-231 et BT20 et par les cellules de la glande submaxillaire, ainsi que l'absence d'une telle sécrétion par les cellules NBEC, et
   - la figure 1C est un graphe donnant la quantité de NGF sécrétée en fonction des cellules testées par ELISA, à savoir les cellules cancéreuses MCF-7, T47D, MDA-MB-231 et BT20, les cellules de la glande submaxillaire et les cellules NBEC,
- la figure 2 concerne des représentations sur la coculture de cellules cancéreuses et de cellules sensibles au NGF, dans laquelle :
   - la figure 2A représente le schéma expérimental de coculture des cellules PC12 (1) se développant en présence de NGF et de cellules cancéreuses (2), et
   - la figure 2B est constituée de photographies représentant la culture de cellules PC12 à titre de témoin (photo 2), la différenciation des cellules PC 12 sous l'influence du NGF (photo 3), la coculture de cellules sensibles au NGF en présence de cellules cancéreuses MCF-7 (photo 4), la culture de cellules cancéreuses MCF-7 en présence d'un anticorps anti-NGF (photo 5), la culture de cellules cancéreuses MCF-7 en présence de l'inhibiteur K252a (photo 6), et la culture de cellules mammaires normales NBEC (photo 7)
- la figure 3 représente des photographies d'immunocytochimie mettant en évidence l'expression de NGF par immunodétection dans du tissu issu de biopsie, dans laquelle les photos A et B concernent le tissu normal, respectivement avec ou sans marquage au NGF, les photos C à F concernant le tissu cancéreux, les photos C et E concernant les coupes sans marquage au NGF (contrôle négatif) et les photos D et F concernant les coupes avec marquage au NGF,
- la figure 4 est relative à des graphes mettant en évidence l'influence d'un partenaire de liaison susceptible de bloquer la prolifération des cellules du cancer du sein sur la croissance cellulaire, dans laquelle :
   - la figure 4A représente un graphe montrant la croissance des cellules MCF-7 en nombre de cellules (10⁴ cellules/disque de 35 mm), soit sans agent de blocage de la prolifération tumorale à titre de témoin, soit en présence de l'anticorps anti-NGF MAB-256, soit en présence de l'inhibiteur K-252a, et
   - les figures 4B et 4C représentent des graphes donnant le nombre de cellules MCF-7 en fonction de la dose de l'anticorps MAB-256 (figure 4B) ou de la dose de l'inhibiteur K-252a (figure 4C) et
- la figure 5 donne des graphes mettant en évidence l'influence d'un anticorps anti-NGF, en fonction du temps, sur le volume tumoral (figure 5A), sur la masse tumorale (figure 5B) et sur le nombre de métastases hépatiques (figure 5C) de cellules de cancer du sein de souris.

### Exemple 1 : Mise en évidence de l'expression du NGF dans des cellules du cancer du sein par RT-PCR

### 1.1 Cellules testées

On a testé 4 lignées de cellules de cancer du sein humain, à savoir les cellules MCF-7, T47D, MDA-MB-231 et BT20, obtenues auprès de la collection ATCC (Rockville, MD) sous les numéros HTB-22, HTB-133, HTB-26 et HTB-29, respectivement.

Ces cellules ont été maintenues dans du milieu essentiel minimal (sel de Earle, BioWhittaker, Belgique) supplémenté avec de l'HEPES 20 mM, 2 g/l de bicarbonate de sodium, 2 mM de L-glutamine, 10% de sérum de veau foetal (FCS), 100 unités/ml de pénicilline-streptomycine, 50 µg/ml de gentamycine, 1 % d'acides aminés non essentiels et 5 µg/ml d'insuline.

A titre de comparaison, on a testé des cellules épithéliales du sein normales (NBEC) obtenues auprès du Département de Chirurgie Plastique de l'Université Médicale de Lille (Pr. Pellerin, France) qu'on a cultivées dans du milieu DMEM/F12 (1/1) (BioWhittaker) contenant 5% de FCS, 10 UI/ml d'insuline, 5 µM de cortisol, 2 ng/ml de EGF, 100 ng/ml de toxine de choléra, 100 ng/ml de pénicilline et 45 µg/ml de gentamycine (milieu B1). Lorsque les cellules ont approché la confluence, on les a placées dans le même milieu, à ceci près qu'on a réduit la concentration en calcium à 20 µM.

On a également utilisé les cellules de glande submaxillaire humaine, obtenues à partir de résidus de dissection hospitaliers, comme source connue de NGF.

### 1.2 RT-PCR

On a isolé l'ARN total des lignées de cellules cancéreuses, des cellules NBEC et de la glande submaxillaire humaine (20 mg de poids) en utilisant le kit Rneasy mini (Qiagen, France). On a mis en oeuvre la rupture et l'homogénéisation de la glande submaxillaire en utilisant un homogénéisateur Rotor-Stator (Bribolyser, Hybaid).

On a quantifié la quantité d'ARN extrait en mesurant l'absorbance à 260 nm et on a recherché la pureté de l'ARN par le rapport d'absorbance à 260 nm et à 280 nm. On a confirmé l'absence de dégradation de l'ARN par électrophorèse de l'ARN sur un gel d'agarose à 1,5% contenant du bromure d'éthidium.

On a réalisé la transcriptase inverse (RT) en ajoutant un mélange réactionnel contenant 2 g d'ARNm total purifié, 1X tampon de réaction, 10 mM de DTT, 400 mM de dNTP chacun, 2,5 M d'oligo(dT), 40 unités de Rnasine et 200 unités de transcriptase inverse de virus de la leucémie murine Moloney à 25 µl de volume réactionnel total. Toutes les réactions ont été incubées à 37°C pendant 1 h, puis inactivées à 95°C pendant 5 min.

La PCR a été mise en oeuvre sur les ADNc après RT ou sur les échantillons d'ARN sans l'étape de RT pour les contrôles négatifs comme suit :

A des tubes de PCR, on a ajouté 5 µl de tampon PCR (200 mM de Tris-HCl, pH 8,4, 500 mM KCl), 10 µl de 15 mM MgCl₂, 1 ml de 10 mM dNTP mix, 1 µl d'ADNc ou d'ARNm total (pour le contrôle négatif), 1 µl de 50 mM des sondes pour le gène *ngf* indiquées ci-dessous, 1 µl de 2,5 unités/µl d'ADN polymérase Taq et de l'eau jusqu'à un volume total de 50 µl.
Sonde sens : 5'-GACAGTGTCAGCGTGTGGGTT-3'
Sonde antisens : 5'-CCCAACACCATCACCTCCTT-3'

Les conditions de PCR étaient les suivantes : après traitement à 95°C pendant 3 min pour dénaturer l'ADNc, on a effectué 30 cycles à 94°C pendant 1 min, 57°C pendant 2 min et 72°C pendant 3 min. On a ensuite incubé les tubes de PCR pour 10 min supplémentaires à 72°C pour l'extension des fragments d'ADNc après le cycle final.

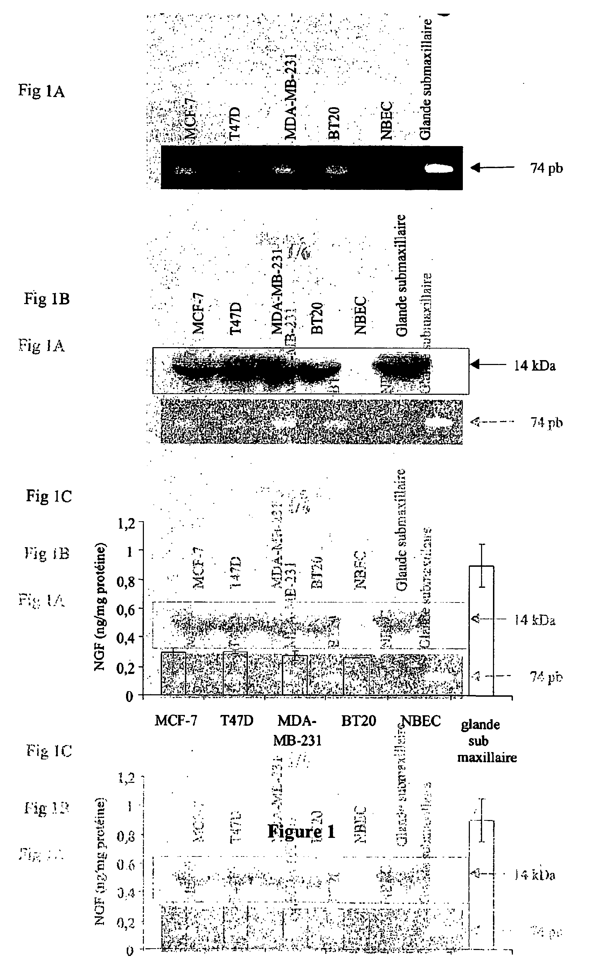

La visualisation du matériel amplifié sous lumière UV par coloration au bromure d'éthidium après électrophorèse sur gel d'agarose est représentée sur la figure 1A. Cette figure montre un produit de transcription du *ngf* de 74 pdb pour les cellules épithéliales cancéreuses et les cellules de la glande submaxillaire (témoin positif), alors que ce produit n'est pas détecté pour les cellules NBEC.

Ce test permet donc de mettre en évidence que les cellules cancéreuses sécrètent du NGF, tandis que les cellules épithéliales correspondantes non cancéreuses n'en sécrètent pas.

### Exemple 2 : Mise en évidence de l'expression du NGF dans des cellules du cancer du sein par Immunoblotting

### 2.1 Cellules testées

Les cellules testées sont celles indiquées dans le point 1.1 ci-dessus.

### 2.2 Immunoblotting

Les cellules subconfluentes, cultivées comme indiqué dans le point 1.1 ci-dessus, ont été rincées et placées dans un milieu sans sérum contenant de la fibronectine et de la transferrine pendant 24 h. Après les avoir privées de nutriments, les cellules ont été collectées par raclage dans du tampon PBS (solution saline tamponnée au phosphate) et on les a soumises à une centrifugation (1000xg, 5 min).

On a traité le culot avec du tampon de lyse (10% SDS, 0,5% de β-mercaptoéthanol, 0,5M Tris HCl pH 6,8, 25% glycérol, 0,5%) et on a mis à bouillir 5 min à 100°C.

Après centrifugation (1000xg, 5 min), on a recherché les protéines des surnageants en utilisant les tests de détermination de protéines de Bio Rad.

On a soumis à SDS-PAGE les lysats provenant de chaque lignée cellulaire et de la glande submaxillaire, on les a transférés sur une membrane de nitrocellulose par électroblotting (200 mA, 45 min).

On a ensuite testé chaque membrane avec un anticorps polyclonal de lapin anti-NGF (sc-548, Santa Cruz Biotechnology, Tebu, France) à 4°C toute la nuit. On a rincé chaque membrane à trois reprises avec du tampon TBS-T, puis on les a incubées à température ambiante pendant 2 h avec un anticorps anti-immunoglobuline de lapin couplé à la peroxydase de raifort (Molecular Probes). On a rincé chaque membrane à quatre reprises avec du tampon TBS-T et on a révélé la réaction en utilisant le kit de chimioluminescence ECL (Amersham Pharmacia Biotech) avec le film AR X-Omat de Kodak.

Là-encore, comme représenté sur la figure 1B, du NGF a été détecté avec les cellules cancéreuses et la glande submaxillaire (bande à 14 kDa), tandis que cette bande était absente pour les cellules non cancéreuses.

Ce résultat confirme celui de l'exemple 1.

### Exemple 3 : Mise en évidence de l'expression du NGF dans des cellules du cancer du sein par ELISA

### 3.1 Cellules testées

Les cellules testées sont celles indiquées dans le point 1.1 ci-dessus.

### 3.2 ELISA

Pour ce test, on a utilisé un test à deux sites pour du β-NGF humain. En résumé, on a revêtu des immunoplaques de 1 µg/ml d'anticorps monoclonal de capture (R&D Systems, UK) dilué dans un tampon de dilution (0,1% BSA, 0,005% Tween 20 dans une solution saline tamponnée au Tris pH 7,3). On a revêtu les puits contrôles de la même quantité d'IgG de souris (R&D Systems). Chaque puits a reçu 100 µl de cette dilution et les plaques ont été scellées et incubées à la température ambiante, toute la nuit.

On a lavé les puits à trois reprises avec 400 µl de PBS, pH 7,4, contenant 0,05% de Tween 20. On a ensuite bloqué les plaques à la température ambiante en utilisant 1 % de sérum albumine bovine, 5% de saccharose et 0,005% de NaN₃ dans le même tampon.

Après 2 h, on a lavé chaque puits, on a appliqué des dilutions de produits inconnus et standards dans un volume de 100 µl, et on a recouvert les plaques d'une bande adhésive et on les a incubées à température ambiante toute la nuit.

On a utilisé du β-NGF humain (R&D Systems) comme échantillon standard à raison de 0,25 à 8 ng/ml dans 100 µl du tampon de dilution.

On a ensuite lavé les plaques de façon extensive et on a ajouté l'anticorps de détection IgG de chèvre spécifique anti-β-NGF humain (BAF-256, R&D Systems) à raison de 50 ng/ml avant incubation pendant 2 h à température ambiante.

Après incubation, on a lavé les immunoplaques à trois reprises avec du tampon de lavage et on a ajouté 100 µl de streptavidine/peroxydase de raifort pour une incubation de 20 min à la température ambiante.

La réaction a commencé par ajout de 100 µl d'une solution substrat pendant 30 min d'incubation à la température ambiante et à l'obscurité.

Après cette durée, on a ajouté 50 µl d'une solution stop (H₂SO₄ 1M) dans chaque puits. On a déterminé la densité optique en 30 min en utilisant un ensemble de lecture de microplaque à 595 ou 450 nm.

Les résultats de densité optique (moyenne de 3 tests indépendants) sont donnés sur la figure 1C. Ils mettent en évidence un niveau similaire de NGF dans toutes les lignées de cellules du cancer du sein testées.

### Exemple 4 : Culture de cellules sensibles au NGF

Pour ce faire, on a cocultivé des cellules qui se développent en présence de NGF, à savoir les cellules PC12, avec des cellules de cancer du sein MCF-7, telles que décrites dans le point 1.1 ci-dessus, ou bien avec des cellules NBEC, telles que décrites dans le point 1.1 ci-dessus, à titre de témoin.

Les cellules PC12 ont été cultivées au préalable à 37°C dans une atmosphère à 5% de CO₂, dans du milieu essentiel minimal modifié de Dulbecco (DMEM) supplémenté avec 3 g/l de bicarbonate de sodium, 4 mM de L-glutamine, 10% de sérum de veau foetal, 5% de sérum de cheval et 100 unités/ml de pénicilline/streptomycine.

La coculture a été réalisée avec des plaques de polycarbonate traitée à la culture tissulaire Transwell® (12 mm de diamètre et 12 µm de taille de pore). Une période d'équilibre initial a été observée en ajoutant les milieux au puits central de la plaque, puis à l'insert du Transwell®, opération suivie d'une incubation à 37°C pendant 3 h.

Les cellules PC12 ont été ensemencées dans des plaques à 12 puits revêtues de collagène pendant 24 h pour assurer une fixation correcte aux plaques de culture. On a ensemencé les cellules MCF-7 ou NCBE à l'intérieur de l'insert du Transwell®, correspondant au compartiment supérieur dans le milieu complet pendant 24 h.

Après ce temps, on a rincé chaque lignée cellulaire à trois reprises avec un milieu dénué de sérum et on les a incubées dans un milieu de retrait. Dans les expériences témoins, le milieu a été supplémenté avec du milieu contenant soit du NGF, soit un inhibiteur de l'activité tyrosine kinase trk (K-252-a, Calbiochem, France), soit de l'anticorps monoclonal de souris anti-β-NGF humain (MAB-256, R&D Systems).

La coculture a été réalisée pendant 48h. Après ce délai, les cellules PC12 ont été fixées avec du méthanol froid (-20°C) pendant 20 min, puis lavées avec une solution saline tamponnée au phosphate (PBS) avant assemblage avec des lamelles couvre-objet et du Glycergel (Dako, France).

On a compté le pourcentage de cellules portant des neurites définies comme des cellules avec un ou plusieurs prolongements neuritiques ayant au moins deux fois la longueur du corps de la cellule. On a également pris des photomicrographies à l'aide d'un microscope à contraste de phase supplémenté avec une caméra digitale optique Olympus.

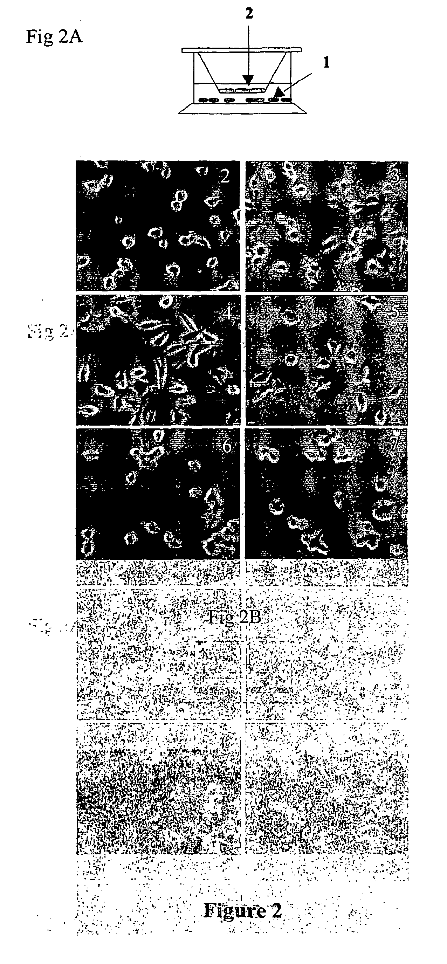

Les résultats sont donnés sur la figure 2, sur laquelle :
- la figure 2A montre le schéma expérimental où (2) correspond aux cellules cancéreuses ou non cancéreuses et (1) correspond aux cellules PC 12,
- la figure 2B donnent des photographies où :
   - les photos 2 et 3 montrent la culture des cellules PC12 à titre de témoin, respectivement sans NGF et en présence du NGF où on observe alors un développement de neurites dû à la différenciation,
   - la photo 4, qui concerne la coculture des cellules MCF-7 et PC12, montre que les cellules PC12 se sont différenciées après 48 h de culture,
   - la photo 5, qui concerne la coculture des cellules MCF-7 et PC12 en présence de l'anticorps anti-NGF, montre une inhibition forte de la différenciation des cellules PC12 du fait de l'anticorps,
   - la photo 6, qui concerne la coculture des cellules MCF-7 et PC12 en présence de l'inhibiteur K-252a, montre une inhibition forte de la différenciation des cellules PC12 du fait de l'inhibiteur, et
   - la photo 7, qui concerne la coculture des cellules normales NBEC et PC12, met en évidence l'absence de différenciation des cellules PC12.

### Exemple 5 : Immunohistochimie pour la détection du NGF dans les biopsies de cancer de sein

### 5.1 Prélèvement et fixation des biopsies

Immédiatement après opération, les biopsies de tissu de cancer du sein humain ou de tissu sain non cancéreux (témoin) ont été fixées par une solution de formol à 10% pendant 24 heures. Après déshydratation par des bains alcooliques croissants (30 à 100%), les échantillons ont été inclus dans la paraffine. Les coupes ont été réalisées à l'aide d'un microtome (coupe fine de 5 micromètres), puis fixées sur des lames revêtues du 3-aminopropyltriéthoxysilane (TESPA, Dako France).

### 5.2 Déparaffinage des lames

On a déparaffiné les lames en trempant les lames dans des bains selon le mode opératoire suivant :
- 2 bains de toluène (10 min),
- 1 bain d'alcool à 100% (10 min),
- 1 bain d'alcool à 95% (10 min),
- 1 bain d'alcool à 70% (10 min),
- 1 bain d'eau (10 min).

On a ensuite conservé les lames à +4°C.

### 5.3 Marquage du NGF

On a rincé les coupes avec du TBS-Tween 0,1% (TBS : 17,54 g de NaCl, 2,42 g de Trisbase, qsp 2 litres d'eau, pH 7,4) pendant 10 min sous agitation. On a réactivé les antigènes par du tampon citrate 0,01M (1,05 g d'acide citrique monohydraté, qsp 500 ml d'eau distillée, pH 6 (NaOH)) 2 fois 5 min dans un bain marie à 95°C. On a procédé à une incubation des lames 5 min sous agitation dans 3% d'eau oxygénée/TBS. On a saturé les lames pendant une heure à température ambiante dans du TBS/3% BSA.

On a ensuite procédé à une incubation des lames 2 h à température ambiante en chambre humide avec 80 µl d'anticorps primaire anti-NGF (Santa Cruz, Tebu France) dilué au 1/50 dans du TBS/3% de BSA. On les a lavées à 3 reprises dans du TBS/Tween pendant 5 min sous agitation.

On a ensuite procédé à une nouvelle incubation des lames 2 h à température ambiante en chambre humide avec 80 µl d'anticorps secondaire de singe anti-IgG de lapin, ledit anticorps ayant été lié à la peroxydase de raifort (Jackson Laboratories, US), dilué au 1/100 dans du TBS/3% de BSA.

On a lavé les lames à trois reprises dans du TBS/Tween pendant 5 min sous agitation, on les a rincées à trois reprises avec du PBS pendant 5 min sous agitation, on les a séchées, puis on les a incubées pendant 10 min avec 100 µl de DAB (sigma fast 3,3-diaminobenzidine ; 1 pastille de DAB + 1 pastille d'urée H2O2 dans 1 ml d'eau).

On a vérifié la coloration sous microscope et on a arrêté dans un bain de PBS. On a effectué une contre coloration pour visualiser les structures mammaires en plongeant dans l'hématoxyline de Harris en solution modifiée (Sigma) pendant 4 s. On a rincé dans de l'eau basique, puis dans un bain de PBS.

On a monté la lame sur glycergel chauffé au bain-marie 50°C.

On a photographié les lames par l'intermédiaire d'une loupe binoculaire (Zeiss).

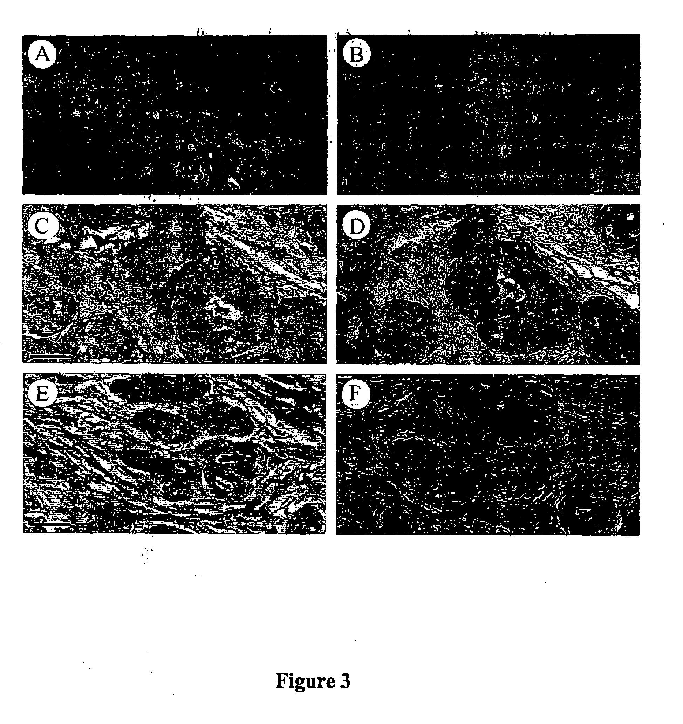

Les photos d'immunohistochimie des coupes ainsi obtenues sont représentées sur la figure 3. Les photos A et B concernent le tissu normal, respectivement avec ou sans marquage au NGF. Les photos C à F concernent le tissu cancéreux, les photos C et E concernant les coupes sans marquage au NGF (contrôle négatif) et les photos D et F concernant les coupes avec marquage au NGF. La barre de calibration représente 250 micromètres pour les photos A, B, E et F ou 600 micromètres pour les photos C et D.

Les résultats montrent clairement que le NGF est détecté par immunohistochimie sur des lames de biopsie dans les cellules épithéliales de cancer du sein, mais pas dans les cellules épithéliales mammaires normales (cellules épithéliales marquées par l'anticorps anti-NGF, et de ce fait colorées, au sein des glandes mammaires tumorales en D et F par rapport à C et E, et non dans les glandes mammaires normales en B par rapport à A).

### Exemple 6: Détection du NGF dans les cellules tumorales par cytométrie en flux

On a décollé les cellules MCF-7 en raclant le fond de la boîte de culture. On a ensuite ajouté 100µl de fixateur (Coulter) avec une incubation de 15 minutes à température ambiante. On a ensuite lavé les cellules à trois reprises avec du PBS et on a centrifugé à 1900 tr/min pendant 5 minutes.

On a ajouté 100 µl de perméabilisateur (Coulter), puis incubé 5 minutes à température ambiante.

On a ajouté 20 µl de l'anticorps monoclonal de souris anti-NGF non marqué (R&D system, dilué au 100^{ème}), puis on a incubé 15 minutes à température ambiante.

On a ensuite lavé les cellules à trois reprises avec du PBS et centrifugé 1900 tr/min pendant 5 minutes.

On a ajouté 20µl du 2^{ème} anticorps anti-souris marqué FITC (R&D system, dilué au 1/50^{ème}) puis incubé 15 minutes à température ambiante et dans l'obscurité.

On a ensuite lavé les cellules à trois reprises avec du PBS et centrifugé 1900 tr/min pendant 5 minutes.

Les cellules ont été reprises dans 500µl de PBS, puis la lecture a été effectuée avec un cytomètre en flux (Beckman Coulter(® EPICS®).

Les résultats obtenus montrent que plus de 30% des cellules de cette lignée cellulaire contiennent du NGF en intracytoplasmique.

Une telle méthode est applicable avec des cellules tumorales isolées d'un échantillon biologique.

### Exemple 7: Influence d'un anticorps anti-NGF et d'un inhibteur pharmacologique sur la croissance des cellules cancéreuses

On a étudié l'effet de l'anticorps de blocage MAB-256 (supra) et de l'inhibiteur K-252a sur la croissance des cellules MCF-7. Pour ce faire, on a ensemencé les cellules à raison de 2x10⁴ cellules/ml dans des boîtes de 35 mm dans du milieu complet. Après avoir atteint 40% de confluence, on a lavé les cellules à deux reprises et on les a laissées sans nutriments dans un milieu dénué de sérum contenant de la fibronectine (2 µg/ml) et de transferrine (30 µg/ml) pendant 3 h. L'heure d'après, on a remplacé le milieu par 2 ml du même milieu contenant l'inhibiteur K-252a ou l'anticorps MAB-26 à différentes concentrations afin d'étudier l'effet de l'inhibiteur pharmacologique ou de l'inhibiteur de blocage sur le niveau basal de croissance.

Après 24, 36, 48 et 72 h de traitement, on a déterminé le nombre de cellules après trypsinisation de la culture monocouche avec une solution de 0,25% trypsine/EDTA en utilisant un hémocytomètre.

On a également utilisé un anticorps monoclonal anti-souris (A4700, SIGMA), c'est-à-dire non pertinent, pour déterminer la spécificité de l'anticorps de blocage.

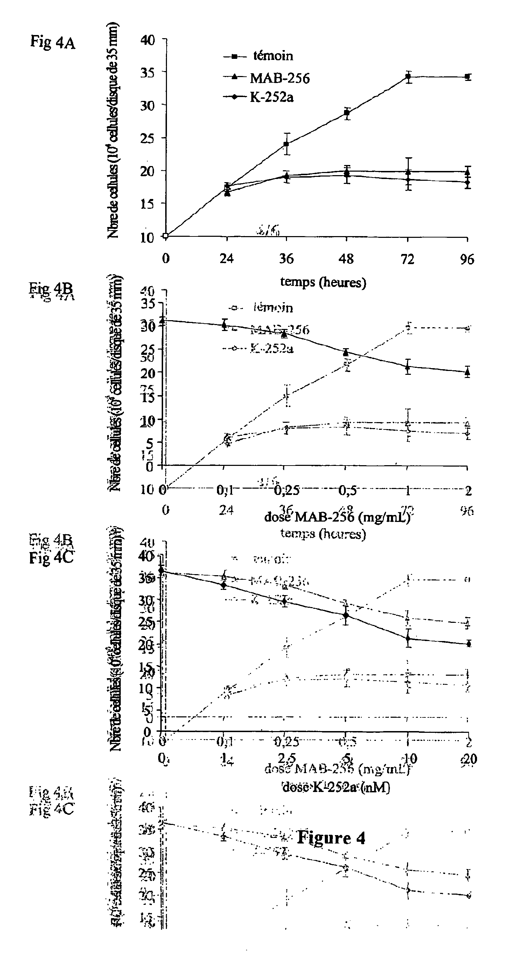

Les résultats sont indiqués sur la figure 4 sur laquelle :
- la figure 4A montre que la croissance des cellules MCF-7 en milieu de base (carrés) diminue en présence d'anticorps (triangles) et d'inhibiteur (losanges),
- les figures 4B et 4C montrent qu'il y a un effet dose de l'anticorps et de l'inhibiteur, respectivement, sur l'effet prolifératif du NGF pour les cellules cancéreuses.

Le NGF peut donc bien être utilisé à titre de cible thérapeutique.

### Exemple 8: Effet des anticorps anti-NGF sur la tumorigénèse des cellules mammaires in vivo

On a étudié la croissance des cellules de cancer du sein MDA-MB-231 comme suit :

On a effectué une injection sous-cutanée dans le flanc de 30 souris SCID (Severe Combined Immuno-Deficience) au temps 0 (J0) de 3×10⁶ cellules MDA-MB-231, issues de cultures *in vitro,* dans 50 microlitres de PBS par souris. On a maintenu les souris en conditions standard d'élevage à température ambiante, avec une alternace jour/nuit et accès sans limitation à la boisson et à la nourriture.

Quatorze , 21 et 28 jours après injection des cellules, on a injecté chez la moitié des souris des anticorps bloquant anti-NGF humains (Oncogene Research Product, VWR France) en périphérie de la tumeur (sous-cutané au niveau du flanc à 1 cm du point d'injection des cellules tumorales) à la concentration de 12,5 microgrammes dans 50 microlitres de tampon PBS par souris.

On a mesuré la taille des tumeurs, sur les souris vivantes, traitées ou non par l'anticorps anti-NGF, avec un pieds à coulisse électronique 21 et 35 jours après l'injection des cellules cancéreuses (J21 et J35, respectivement). Le volume de la tumeur a été calculé avec la formule suivante : 0,5236×longueur (mm)xlargeur (mm)x(longueur+largeur)/2.

On a également mesuré le poids des tumeurs à J35 sur ces mêmes souris, après sacrifice de l'animal, à l'aide d'une balance de précision électronique.

On a enfin compté macroscopiquement le nombre de métastases sur leur foie.

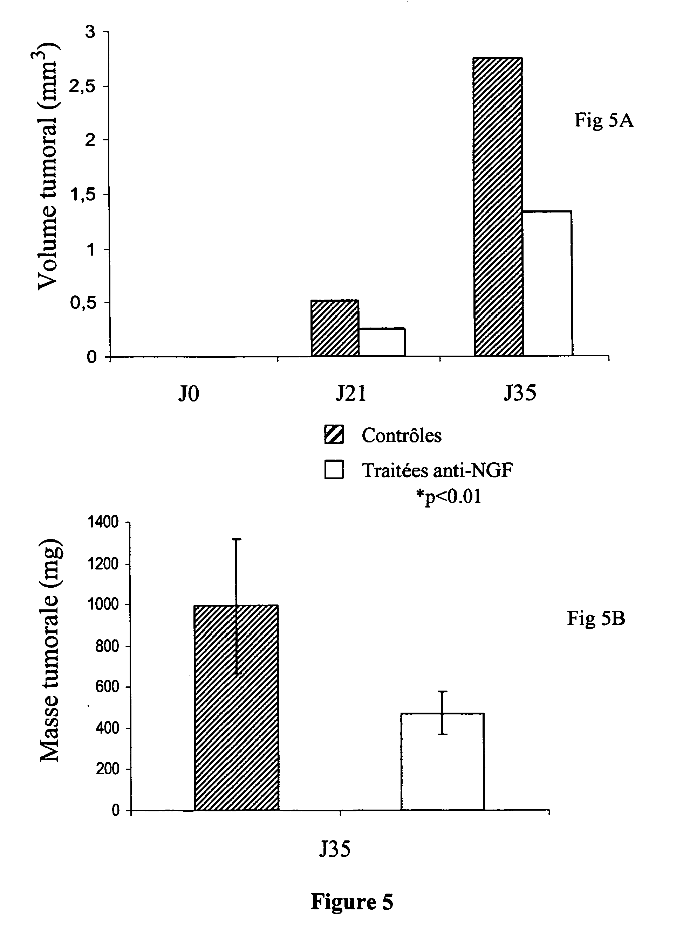

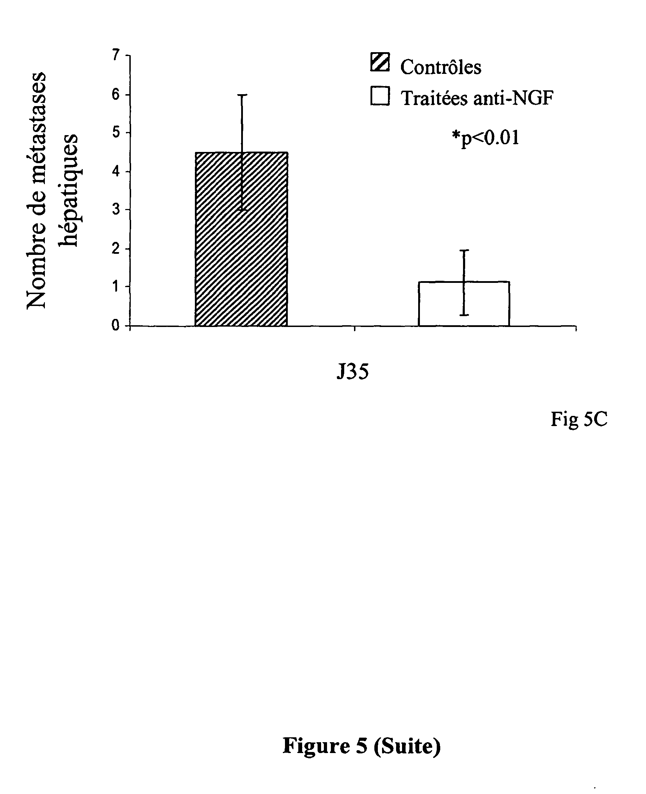

Les résultats respectifs des mesures ci-dessus (moyenne) sont indiqués sur les graphes de la figure 5 où l'histogramme rayé correspond aux souris non traitées par l'anticorps anti-NGF (contrôles) et l'histogramme non rempli correspond aux souris traitées par ledit anticorps.

Ces graphes mettent en évidence que les traitements par un anti-NGF entraîne une diminution de la masse et du volume tumoraux, ainsi que le nombre de métastases dans le cadre du cancer du sein.

## Revendications

1. Procédé de diagnostic *in vitro* du cancer du sein, **caractérisé en ce qu'**il consiste à déterminer la présence de NGF dans un échantillon biologique issu d'un patient suspecté d'être atteint du cancer du sein.

2. Procédé selon la revendication 1, **caractérisé en ce que** la présence de NGF est mise en évidence par détection directe du NGF dans ledit échantillon biologique.

3. Procédé selon la revendication 2, **caractérisé en ce que** la détection du NGF est mise en oeuvre par un test immunologique ou par spectrométrie de masse.

4. Procédé selon l'une des revendications 2 ou 3, **caractérisé en ce que** ledit échantillon biologique est constitué de fluide biologique ou d'un tissu provenant de la biopsie de la tumeur ou de métastases.

5. Procédé selon la revendication 4, **caractérisé en ce que** l'échantillon biologique est constitué d'un tissu provenant de la biopsie de la tumeur ou des métastases du patient.

6. Procédé selon la revendication 4, **caractérisé en ce que** l'échantillon biologique est constitué de fluide biologique, de préférence préalablement traité pour isoler les cellules tumorales circulantes contenues dans ledit fluide.

7. Procédé selon la revendication 6, **caractérisé en ce que** les cellules tumorales circulantes sont ensuite cultivées en conditions telles qu'elles sécrètent du NGF.

8. Procédé selon l'une des revendications 6 ou 7, **caractérisé en ce que** les cellules tumorales circulantes sont également cultivées dans des conditions permettant le blocage du NGF à l'intérieur desdites cellules.

9. Procédé selon la revendication 1, **caractérisé en ce que** la détection de NGF est mise en évidence par culture, en présence dudit échantillon biologique, de cellules sensibles au NGF.

10. Procédé selon la revendication 9, **caractérisé en ce que** ledit échantillon biologique est constitué d'un échantillon de fluide biologique, de préférence préalablement traité pour isoler les cellules tumorales circulantes contenues dans ledit fluide.

11. Procédé selon la revendication 10, **caractérisé en ce que** les cellules tumorales circulantes sont ensuite cultivées en conditions telles qu'elles sécrètent du NGF.

12. Procédé selon la revendication 1, **caractérisé en ce que** la détection de NGF est mise en évidence par détection de l'ARNm du NGF dans ledit échantillon biologique.

13. Procédé selon la revendication 12, **caractérisé en ce que** ledit échantillon biologique est constitué d'un échantillon de fluide biologique ou d'un tissu provenant de la biopsie de la tumeur ou des métastases du patient.

14. Procédé selon la revendication 13, **caractérisé en ce que** l'échantillon biologique est constitué d'un tissu provenant de la biopsie de la tumeur ou des métastases du patient.

15. Procédé selon la revendication 13, **caractérisé en ce que** l'échantillon biologique est constitué de fluide biologique, de préférence préalablement traité pour isoler les cellules tumorales circulantes contenues dans ledit fluide.

16. Utilisation du procédé selon l'une quelconque des revendications 1 à 15 dans le diagnostic précoce, le dépistage, le suivi thérapeutique, le pronostic et le diagnostic des rechutes dans le cadre du cancer du sein.

## Claims

1. A method for the *in vitro* diagnosis of breast cancer, **characterized in that** it consists in determining the presence of NGF in a biological sample derived from a patient suspected of suffering from breast cancer.

2. The method as claimed in claim 1, **characterized in that** the presence of NGF is demonstrated by direct detection of NGF in said biological sample.

3. The method as claimed in claim 2, **characterized in that** detection of NGF is carried out by means of an immunoassay or by mass spectrometry.

4. The method as claimed in either of claims 2 and 3, **characterized in that** said biological sample consists of biological fluid or of a tissue originating from the biopsy of the tumor or of metastases.

5. The method as claimed in claim 4, **characterized in that** the biological sample consists of a tissue originating from the biopsy of the tumor or of the metastases of the patient.

6. The method as claimed in claim 4, **characterized in that** the biological sample consists of biological fluid, preferably pretreated in order to isolate the circulating tumor cells contained in said fluid.

7. The method as claimed in claim 6, **characterized in that** the circulating tumor cells are then cultured under conditions such that they secrete NGF.

8. The method as claimed in either of claims 6 and 7, **characterized in that** the circulating tumor cells are also cultured under conditions that block the NGF inside said cells.

9. The method as claimed in claim 1, **characterized in that** the detection of NGF is demonstrated by culturing NGF-sensitive cells in the presence of said biological sample.

10. The method as claimed in claim 9, **characterized in that** said biological sample consists of a sample of biological fluid, preferably pretreated in order to isolate the circulating tumor cells contained in said fluid.

11. The method as claimed in claim 10, **characterized in that** the circulating tumor cells are then cultured under conditions such that they secrete NGF.

12. The method as claimed in claim 1, **characterized in that** the detection of NGF is demonstrated by detecting the NGF mRNA in said biological sample.

13. The method as claimed in claim 12, **characterized in that** the said biological sample consists of a sample of biological fluid or of a tissue originating from the biopsy of the tumor or of the metastases of the patient.

14. The method as claimed in claim 13, **characterized in that** the biological sample consists of a tissue originating from the biopsy of the tumor or of the metastases of the patient.

15. The method as claimed in claim 13, **characterized in that** the biological sample consists of biological fluid, preferably pretreated in order to isolate the circulating tumor cells contained in said fluid.

16. The use of the method as claimed in any one of claims 1 to 15 in early diagnosis, screening, therapeutic follow-up, prognosis and the diagnosis of relapse in the case of breast caner.

## Patentansprüche

1. In-vitro-Verfahren für die Diagnose von Brustkrebs, **dadurch gekennzeichnet, daß** es darin besteht, daß man das Vorliegen von NGF in einer biologischen Probe von einem Patienten mit Verdacht auf Brustkrebs bestimmt.

2. Verfahren nach Anspruch 1, **dadurch gekennzeichnet, daß** das Vorliegen von NGF durch direkte Bestimmung von NGF in der biologischen Probe nachgewiesen wird.

3. Verfahren nach Anspruch 2, **dadurch gekennzeichnet, daß** die NGF-Bestimmung durch einen immunologischen Test oder durch Massenspektrometrie durchgeführt wird.

4. Verfahren nach einem der Ansprüche 2 oder 3, **dadurch gekennzeichnet, daß** die biologische Probe aus einer biologischen Flüssigkeit oder einem Gewebe aus der Biopsie des Tumors oder von Metastasen besteht.

5. Verfahren nach Anspruch 4, **dadurch gekennzeichnet, daß** die biologische Probe aus einem Gewebe aus der Biopsie des Tumors oder der Metastasen des Patienten besteht.

6. Verfahren nach Anspruch 4, **dadurch gekennzeichnet, daß** die biologische Probe aus biologischer Flüssigkeit besteht, die vorzugsweise zuvor behandelt wurde, um die zirkulierenden Tumorzellen, die in dieser Flüssigkeit enthalten sind, zu isolieren.

7. Verfahren nach Anspruch 6, **dadurch gekennzeichnet, daß** die zirkulierenden Tumorzellen anschließend unter Bedingungen kultiviert werden, unter denen sie NGF sezernieren.

8. Verfahren nach einem der Ansprüche 6 oder 7, **dadurch gekennzeichnet, daß** die zirkulierenden Tumorzellen auch unter Bedingungen kultiviert werden, die die Hemmung von NGF innerhalb dieser Zellen gestatten.

9. Verfahren nach Anspruch 1, **dadurch gekennzeichnet, daß** die NGF-Bestimmung durch Kultivieren von gegenüber NGF sensitiven Zellen in Gegenwart der biologischen Probe nachgewiesen wird.

10. Verfahren nach Anspruch 9, **dadurch gekennzeichnet, daß** die biologische Probe aus biologischer Flüssigkeit besteht, die vorzugsweise zuvor behandelt wurde, um die zirkulierenden Tumorzellen, die in dieser Flüssigkeit enthalten sind, zu isolieren.

11. Verfahren nach Anspruch 10, **dadurch gekennzeichnet, daß** die zirkulierenden Tumorzellen anschließend unter Bedingungen kultiviert werden, unter denen sie NGF sezernieren.

12. Verfahren nach Anspruch 1, **dadurch gekennzeichnet, daß** die NGF-Bestimmung durch Bestimmung der mRNA des NGF in der biologischen Probe nachgewiesen wird.

13. Verfahren nach Anspruch 12, **dadurch gekennzeichnet, daß** die biologische Probe aus einer biologischen Flüssigkeit oder einem Gewebe aus der Biopsie des Tumors oder von Metastasen des Patienten besteht.

14. Verfahren nach Anspruch 13, **dadurch gekennzeichnet, daß** die biologische Probe aus einem Gewebe aus der Biopsie des Tumors oder der Metastasen des Patienten besteht.

15. Verfahren nach Anspruch 13, **dadurch gekennzeichnet, daß** die biologische Probe aus biologischer Flüssigkeit besteht, die vorzugsweise zuvor behandelt wurde, um die zirkulierenden Tumorzellen, die in dieser Flüssigkeit enthalten sind, zu isolieren.

16. Verwendung des Verfahrens nach einem der Ansprüche 1 bis 15 in der Frühdiagnose, der Erkennung, der Nachsorge, der Prognose und der Diagnose von Rückfällen im Zusammenhang mit Brustkrebs.
